# FASCICULE DE BREVET EUROPEEN

(11) **EP 1 983 919 B1**
(45) Date de publication et mention de la délivrance du brevet: **23.06.2010**
(21) Numéro de dépôt: 07730877.3
(22) Date de dépôt: 29.01.2007
(51) Int. Cl.: A61B 18/14

(54) **INSTRUMENT PERMETTANT DE RETIRER DES TIQUES FIXEES SUR LA PEAU**
INSTRUMENT ZUM ENTFERNEN VON ZECKEN, DIE SICH AN DIE HAUT GEHEFTET HABEN
INSTRUMENT FOR REMOVING TICKS THAT ARE FIXED TO THE SKIN

(30) Priorité: 31.01.2006 FR 0600854
(43) Date de publication de la demande: 29.10.2008
(73) Titulaire: ACTIPHARM SA, Canton de Genève (CH)
(72) Inventeur: Schaumburg, Raymond, 63270 Issertaux (FR)
(74) Mandataire: Myon, Gérard Jean-Pierre
(86) Numéro de dépôt international: PCT/FR2007/000159
(87) Numéro de publication internationale: WO 2007/088264

(56) Documents cités:
- DE-A1- 19 913 210
- US-A- 4 213 460
- US-A- 5 374 274

## Description

L'invention a trait à un instrument permettant de retirer les tiques fixées sur la peau d'un animal ou d'un être humain.

Ces insectes se fixent sur la peau des animaux ou des êtres humains par l'intermédiaire d'un rostre pourvu d'ardillons et situé à l'extrémité de la tête. Par ce rostre, l'insecte prélève du sang sur l'être vivant sur lequel il est fixé. Il injecte également des produits toxiques pour l'hôte. Ces produits toxiques sont responsables de maladies, en particulier chez l'homme de la maladie de Lyme. Il est donc nécessaire de retirer le plus tôt possible l'insecte de l'hôte, en évitant que le rostre ou une partie de celui-ci reste dans la peau.

On connaît par US-A-4 976 718 une pince dont les extrémités concaves dentées emprisonnent l'animal, le pincent au niveau de la tête et le maintiennent lors de son extraction. US-A-5 116 347 décrit une pince dont les extrémités en forme de cuillère pincent l'animal au niveau de la tête pour son extraction. Ces différents dispositifs ne permettent pas d'extraire la tique en étant sûr qu'aucune partie du rostre ne reste dans la peau. Par ailleurs, le mode d'utilisation de ces différents dispositifs induit une compression de l'animal lors de l'extraction. Cette compression, lorsqu'elle a lieu au niveau de la tête, provoque l'injection de produits toxiques par le rostre juste avant l'extraction. Lorsqu'il y a compression au niveau abdominal, cela provoque la dissémination de produits toxiques dans l'environnement et/ou d'oeufs lorsque la tique est prête à pondre. On connaît également par US-A-4 748 767 un instrument, en forme de cloche, dont les bords recouvrent l'insecte et adhérent à la peau de l'hôte. Cette cloche est pourvue en partie supérieure d'orifices permettant l'injection d'une substance destinée à neutraliser la tique et/ou à faire le vide dans la cloche. Ce dispositif est peu aisé à mettre en oeuvre, car il faut assurer son étanchéité par rapport à la peau, ce qui dans le cas d'un animal pourvu d'un pelage n'est pas aisé. Il nécessite également des systèmes d'injection de produit et/ou d'extraction d'air que l'on branche sur le dispositif. Par ailleurs, lorsque la tique est décrochée, en enlevant la ventouse, on risque de laisser tomber et de perdre la tique, ce qui n'est pas satisfaisant au niveau de l'hygiène.

DE-A-19913210 décrit une pince selon le préambule de la revendication 1 aux extrémités arrondies et pourvues de conducteurs électriques permettant d'électrocuter une tique. Cette pince est relativement encombrante et nécessite une alimentation en énergie par une pile. Les extrémités des branches de l'instrument sont ouvertes latéralement. De plus, la conformation des extrémités ne permet pas de garantir que l'animal sur lequel se trouve la tique ne recevra pas également une décharge électrique.

US-A-4 213 460 concerne une pince aux extrémités en demi sphères pourvues d'une résistance électrique. Cette pince, également encombrante et nécessitant une alimentation en énergie par une pile, peut provoquer des brûlures sur la peau de l'animal.

C'est à ces inconvénients qu'entend plus particulièrement remédier l'invention en proposant un instrument permettant de retirer les tiques, aisé à mettre en oeuvre dans des conditions d'hygiène satisfaisantes, évitant la dispersion de produits toxiques et/ou des oeufs lors de l'extraction et assurant l'extraction de la tique sans laisser subsister une partie du rostre dans l'hôte, sans injection de produits toxiques lors de l'extraction, d'un encombrement réduit, sans risque pour l'animal.

A cet effet, l'invention a pour objet un instrument permettant de retirer des tiques fixées sur la peau d'un animal ou d'un être humain, cet instrument comportant deux parties mobiles l'une par rapport à l'autre, ces parties comprenant chacune un organe adapté pour définir avec l'autre organe un volume clos de maintien sans compression du corps et de la tête d'une tique, ce volume clos étant pourvu d'un moyen de neutralisation létal pour la tique **caractérisé en ce que** le moyen de neutralisation est raccordé à un générateur piézoélectrique adapté pour délivrer au moyen de neutralisation une tension électrique provoquant un choc électrique létal pour la tique.

En disposant la tique, préalablement à son extraction, dans un volume clos de maintien, on évite la perte de celle-ci après extraction, tout en évitant des compressions sur le corps et/ou la tête de l'insecte. Un choc électrique létal dans ce volume clos permet d'agir rapidement sur l'insecte, sans toucher l'animal. Celui-ci, une fois mort, se détache naturellement sans laisser subsister une partie du rostre dans la peau de l'hôte et sans libération de produits toxiques et/ou d'oeufs.

Selon des aspects avantageux mais non obligatoires de l'invention, l'instrument peut incorporer une ou plusieurs des caractéristiques suivantes :
- La tension électrique délivrée au moyen de neutralisation par le générateur piézoélectrique est d'au moins 17 000 volts.
- Le générateur piézoélectrique est équipé d'un dispositif adapté pour éviter une baisse de la tension électrique délivrée au moyen de neutralisation avec le temps. Ce dispositif comprend une capacité électrique déchargeant dans une bobine électrique adaptée pour amplifier et linéariser la tension fournie par le générateur piézoélectrique.
- Le générateur piézoélectrique est actionné par un déplacement d'un capuchon coiffant une extrémité du corps de l'instrument.
- Les organes définissent chacun une demi sphère dont l'une est fixée à une extrémité du corps de l'instrument, l'autre demi sphère étant fixée à une extrémité d'un bras mobile par rapport au corps de l'instrument.
- L'un des organes est pourvu d'un décrochement formant un passage pour la tête d'une tique.
- Le générateur piézoélectrique alimente deux conducteurs électriques disposés de part et d'autre du passage, les conducteurs étant adaptés pour réaliser un arc électrique lorsque les organes forment le volume clos.
- Le générateur piézoélectrique alimente, dans chaque organe, au moins un conducteur électrique disposé sur la face interne, les conducteurs étant adaptés pour réaliser un arc électrique lorsque les organes forment le volume clos.
- Au moins un organe est réalisé en un matériau transparent.
- L'organe transparent forme une loupe.
- Le corps de l'instrument porte l'un des organes alors que l'autre organe est porté par un bras monté mobile sur le corps et en ce que le corps est équipé d'un moyen de rappel en position du bras mobile vers une position où les organes définissent ensemble le volume clos de maintien.de la tique.

L'invention sera mieux comprise et d'autres avantages de celle-ci apparaîtront plus clairement à la lecture de la description qui va suivre d'un instrument conforme à l'invention, donnée uniquement à titre d'exemple et faite en se référant aux dessins annexés dans lesquels :
- la figure 1 est une vue en perspective d'un instrument conforme à l'invention; ses parties mobiles étant en position ouverte, avant utilisation,
- la figure 2 est une vue de l'instrument de la figure 1 en position de d'utilisation, une tique étant schématiquement représentée en transparence dans le volume,
- la figure 3 est une vue en perspective à plus grande échelle d'un bras mobile de l'instrument portant une demi sphère de maintien du corps d'une tique,
- la figure 4 est une vue en perspective, à la même échelle que la figure 1, du corps de l'instrument avec une autre demi sphère de maintien du corps d'une tique et
- la figure 5 est une vue de côté du corps de l'instrument représenté à la figure 4, la source d'énergie étant schématisée en pointillés..

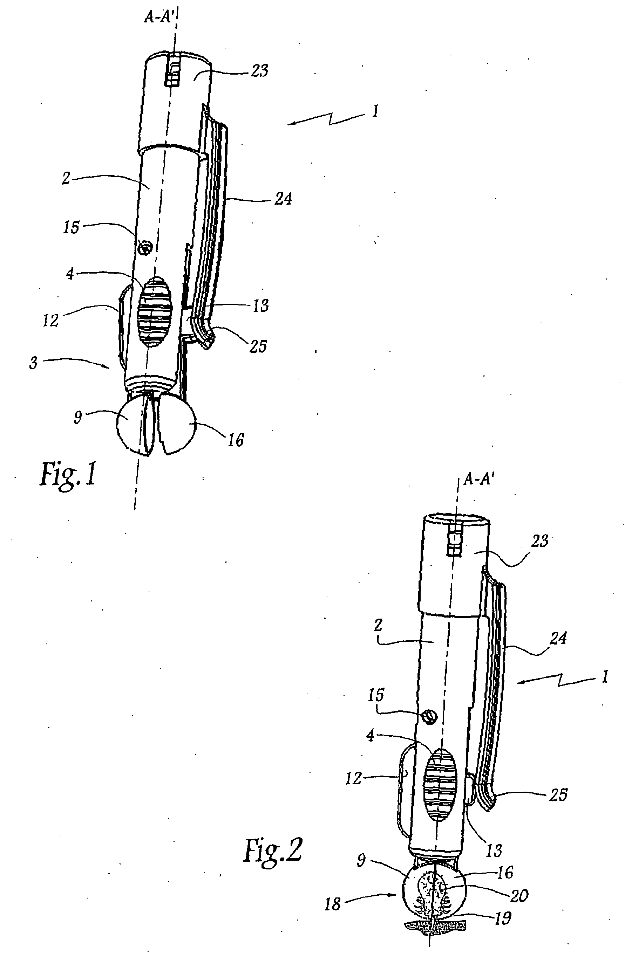

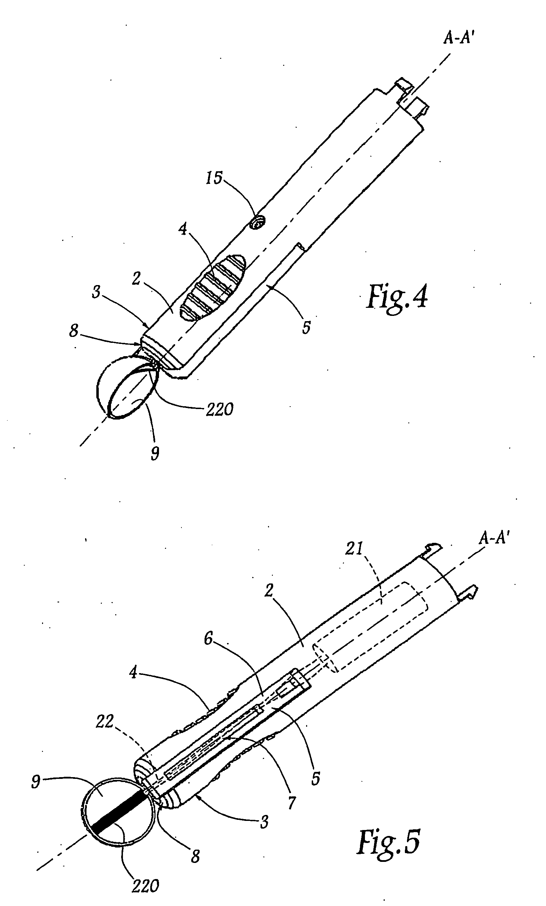

L'instrument 1 représenté aux figures 1 et 2 comprend un corps principal 2 globalement tubulaire et réalisé en un matériau rigide, léger, insensible aux agressions physiques et chimiques. Avantageusement, le corps principal est réalisé en un matériau à base de polymères. Au voisinage d'une première extrémité 3 de ce corps 2 tubulaire, dont la longueur est globalement comprise entre 9 centimètres et 11 centimètres et le diamètre compris entre 1 centimètre et 1,5 centimètre, sont disposés deux lamages 4, ovales et rainurés, disposés diamétralement sur le corps et permettant une prise en main aisé par l'utilisateur. Sur globalement la moitié de sa longueur, entre les lamages 4, le corps 2 est équipé d'une ouverture allongée ou fente 5 parallèle à un axe longitudinal A-A' du corps 2. Cette fente 5, visible notamment à la figure 5, permet d'accéder à l'alésage interne du corps. La paroi du fond 6 de cette fente 5, est pourvue d'un orifice 7 allongé, de longueur inférieure à celle de la fente 5. Cet orifice 7 est traversant et orienté également selon une direction parallèle à l'axe A-A'. La fente 5 a globalement une section transversale configurée en U.

La fente 5 débouche sur la face terminale 8 de l'extrémité 3. Cette extrémité 3 est équipée d'un organe 9, constitutif d'une moitié d'un volume clos de maintien de la tique. Cet organe 9 est configuré en une demi sphère de rayon compris entre 10 et 11 millimètres. Dans un mode de réalisation non représenté, cet organe n'est pas configuré en demi sphère mais en fuseau ou en une autre figure géométrique, par exemple un parallélépipède.

L'ouverture de la demi sphère 9 est située au voisinage du fond 6 de la fente 5. La face externe de la paroi de la demi sphère 9 est tangente à la face terminale 8. La demi sphère 9 s'étend vers l'extérieur du corps 2, sensiblement dans le prolongement de l'axe A-A'.

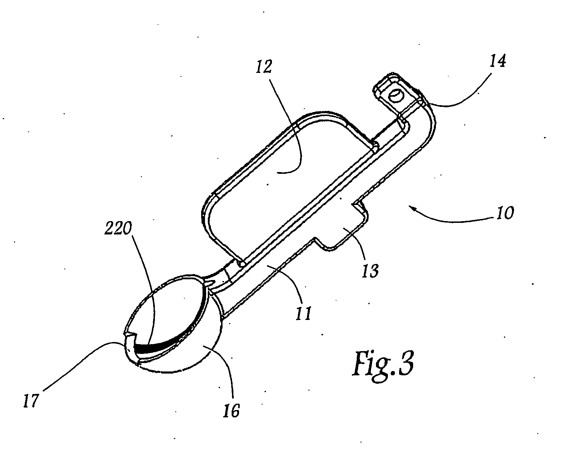

La fente 5 et l'orifice 7 sont adaptés pour recevoir un bras mobile 10 représenté à la figure 3. Ce bras 10 comprend une partie principale, globalement en forme de barre 11 rectangulaire. Cette barre 11 est équipée, sur un côté, d'un voile 12 et, sur un côté opposé, d'un relief 13. Le voile 12 et le relief 13 s'étendent de part et d'autre de la barre 11. Une extrémité de la barre 11 est pourvue d'une patte 14 coudée et percée permettant le passage d'un axe 15 de rotation du bras 10 lorsque l'orifice de cette patte 14 est en regard avec des orifices traversants ménagés dans le corps 2 de l'instrument, au voisinage de l'extrémité fermée de la fente 5. L'extrémité opposée du bras 10 est équipée d'un organe 16 conformé en demi sphère et formant l'autre moitié du volume clos de maintien de la tique. Cette demi sphère formée par l'organe 16 est similaire à la demi sphère formée par l'organe 9. L'organe 16 est pourvu d'un décrochement périphérique 17 diamétralement opposé à son point de fixation sur la barre 11. La fixation de l'organe 16 est faite de manière à ce que la face externe de la paroi de son ouverture soit au voisinage d'un coté de la barre 11 et orientée en direction du voile 12. Dans un mode de réalisation non représenté, le décrochement est ménagé sur l'organe 9. En variante, il est ménagé sur les deux organes 9, 16.

Lorsqu'on insère le bras 10 dans la fente 5 en positionnant le relief 13 dans l'orifice 7, les deux organes 9 et 16 sont situés en regard l'un de l'autre, leur concavité se faisant face. Grâce à l'axe de rotation 15, le bras 10 est solidaire du corps 2 et mobile en pivotement par rapport à celui-ci. Le mouvement du bras 10 permet de rapprocher ou d'écarter les organes 9 et 16 l'un de l'autre. Ainsi, on réalise un instrument 1 en forme de pince, équipé de deux demi volumes 9, 16 à une extrémité qui, lorsqu'ils sont adjacents, définissent un volume clos de maintien d'une tique, ce volume 18 étant configuré en sphère. Ce volume 18 présente une ouverture formée par le décrochement 17. Ce décrochement 17 permet le passage de la tête 19 d'une tique 20, sans compression.

Le corps 2 de l'instrument abrite une source d'énergie, dans l'exemple un générateur piézoélectrique 21, représenté uniquement à la figure 5 pour la clarté du dessin. L'utilisation d'un générateur piézoélectrique permet de réaliser un instrument de faible encombrement, léger et ne nécessitant pas de source d'énergie telle qu'une pile. Ce générateur est relié par un fil conducteur 22 à l'extrémité de la fente 5, au niveau de l'ouverture de l'organe 9. Ce générateur 21 est équipé à une extrémité d'un interrupteur de mise en marche, non représenté.

Dans le mode de réalisation illustré, chacun des organes 9, 16 est équipé d'au moins un conducteur 220 disposé sur sa face interne. Ces conducteurs 220 sont réalisés à partir de feuilles d'acier inoxydable, d'environ un dixième de millimètre d'épaisseur, fixées sur les faces internes des organes 9 et 16, par exemple par collage haute fréquence, par revêtement électrolytique ou par dépôt en phase vapeur. Le conducteur 220 de l'organe 9 est relié au fil conducteur 22 provenant du générateur piézoélectrique 21. Lorsque les deux organes 9, 16 sont refermés l'un contre de l'autre, leurs conducteurs 220 définissent un cercle sur la face interne du volume 18.

Dans un mode de réalisation non représenté, les conducteurs 220 sont disposés sur la face interne d'un organe en demi sphère, en l'espèce l'organe 16, de part et d'autre du passage 17. De cette manière, on dispose les moyens de neutralisation au plus près de la tête de l'animal. Les conducteurs 220 sont adaptés pour réaliser un arc électrique au niveau de la tête 19 de la tique 20 lorsque les demi-sphères 9, 16 forment le volume clos 18.

En variante, les conducteurs sont des fils électriques.

A son extrémité opposée à celle équipée de l'organe 9, le corps 2 de l'instrument est coiffé par un capuchon 23, déplaçable en translation selon une direction parallèle à l'axe A-A'. Ce capuchon 23 est équipé d'une agrafe 24 dont l'extrémité libre 25 vient en appui sur le relief 13 du bras 10 lorsque ce relief 13 est poussé à l'extérieur de l'orifice 7 par une pression manuelle sur le voile 12 placé de l'autre côté. Cette pression de la part de l'utilisateur tend à repousser le bras 10 hors de la fente 5. Ce mouvement génère donc un éloignement mutuel des demi sphères formées par les organes 9 et 16, ce qui tend à une ouverture maximale de l'instrument 1, notamment avant son utilisation.

Le relief 13 est pourvu à son extrémité d'une rainure formant un logement à un relief complémentaire porté par l'agrafe 24. Ainsi, le mouvement d'éloignement du bras 10 du corps 2 est guidé, l'agrafe 24 restant en position sur le relief 13.

Le relâchement de la pression sur le voile 12 permet le rappel en position du bras 10 par appui de l'agrafe 24 sur le relief 13. Ceci a pour effet de rapprocher mutuellement les organes 9 et 16. Si nécessaire, ce mouvement est complété par une pression de l'utilisateur sur l'agrafe 24. Ainsi, d'une part, en jouant sur l'effet ressort de l'agrafe 24 et, d'autre part, en appuyant sur le voile 12, on réalise une ouverture et une fermeture efficace et aisée de l'instrument, les organes 9 et 16 formant, en position rapprochée, un volume clos 18 autour de la tique, sans compression sur celle-ci.

Avantageusement, au moins un organe 9 ou 16 est réalisé en un matériau transparent permettant de visualiser l'extraction de la tique. L'organe transparent forme avantageusement une loupe permettant de visualiser la tique en place dans le volume clos 18.

Un déplacement longitudinal du capuchon 23, par pression manuelle sur ce dernier, permet d'actionner l'interrupteur du générateur 21. Le générateur piézoélectrique 21 délivre alors une tension électrique, voisine de 17 000 volts, entre les conducteurs 220 disposés à l'intérieur des organes 9 et 16. Cette tension est suffisante pour générer un choc électrique, au voisinage de la tête 19 de l'insecte, qui neutralise le système neurologique et enzymatique de ce dernier. L'utilisation de demi sphères pour définir un volume clos dans lequel cette tension est délivrée permet de réaliser une cage de Faraday qui évite que l'animal qui porte la tique ressente les effets de la tension. En d'autres termes, le choc électrique n'est perçu que par la tique.

Il est connu qu'un générateur piézoélectrique délivre une tension électrique qui diminue avec le temps. Pour maintenir à une valeur élevée, et au minimum de 17 000 volts, cette tension électrique, on équipe le générateur 21 d'un dispositif d'amplification et de linéarisation de la tension délivrée aux conducteurs 220. Ce dispositif, non représenté, comprend une capacité électrique qui se décharge dans une bobine. Cette dernière étant reliée à un conducteur 220, la tension délivrée par le générateur 21 est amplifiée, en l'espèce environ 12 fois, et linéarisée. Ainsi même une baisse de la tension de sortie du générateur 21 dans le temps ne provoque pas une baisse de l'efficacité de l'instrument, la tension électrique appliquée au niveau des conducteurs 220 restant supérieure ou égale à 17 000 volts.

On réalise ainsi un moyen de neutralisation létal pour la tique 20. Les systèmes neurologiques et enzymatiques commandent des réflexes d'ancrage du rostre et d'injection de produits toxiques dans l'hôte. On assure, par leur neutralisation, le décrochage naturel de la tique et on évite un phénomène de libération de produits toxiques par le rostre et/ou l'abdomen, puisque le corps de l'animal n'est pas soumis à une compression. La tique 20 ainsi tuée, ou pour le moins assommée, peut donc, en toute hygiène et sans aucun contact avec l'utilisateur ou l'hôte, être mise dans un système de collecte ou détruite, par exemple par le feu.

Avec une tension voisine de 17 000 volts, le choc électrique est suffisant pour affecter immédiatement le système neurologique de la tique, des essais ayant montré une neutralisation du système neurologique à partir d'une tension de 10 000 volts. Des essais pratiqués sur une centaine de tiques de sept espèces différentes, accrochées sur six chiens, ont montrés que 100 % des tiques ont été extraites avec leur rostre, en une seule fois. Parmi celles-ci 11 % présentaient un mouvement réflexe des pattes après leur extraction mais, toutes étaient mortes dans les trois minutes suivant le choc. Lors de ces essais, aucune tique n'a été perdue ni tombée au sol et aucune n'a relâché de produits toxiques et/ou d'oeufs.

Dans un autre mode de réalisation non illustré, le moyen de rappel en position du bras mobile est complété ou remplacé par un ressort. De même, la mise en marche du moyen de neutralisation peut se faire par un poussoir placé latéralement sur le corps de l'instrument ou par rotation d'une partie du corps de l'instrument.

## Revendications

1. Instrument permettant de retirer des tiques fixées sur la peau d'un animal ou d'un être humain, ledit instrument comportant un corps (2) et deux parties mobiles l'une par rapport à l'autre, lesdites parties (2, 10) comprenant chacune un organe (9,16) adapté pour définir avec l'autre organe (16, 9) un volume clos (18) de maintien sans compression du corps et de la tête (19) d'une tique (20), ledit volume (18) étant pourvu d'un moyen neutralisation létal (220) pour ladite tique (20) raccordé à un générateur piézoélectrique (21) adapté pour délivrer au moyen de neutralisation une tension électrique provoquant un choc électrique létal pour la tique, **caractérisé en ce que** le générateur piézoélectrique (21) est inséré dans le corps (2) de l'instrument et **en ce qu'**il est actionné par un déplacement d'un capuchon (23) coiffant une extrémité du corps (2) de l'instrument.

2. Instrument selon la revendication 1, **caractérisé en ce que** la tension électrique délivrée au moyen de neutralisation (220) par le générateur piézoélectrique (21) est d'au moins 17 000 volts.

3. Instrument selon l'une des revendications précédentes, **caractérisé en ce que** le générateur piézoélectrique (21) est équipé d'un dispositif adapté pour éviter une baisse de la tension électrique délivrée au moyen de neutralisation (220) avec le temps.

4. Instrument selon la revendication 3, **caractérisé en ce que** ledit dispositif comprend une capacité électrique déchargeant dans une bobine électrique adaptée pour amplifier et linéariser la tension fournie par le générateur piézoélectrique (21).

5. Instrument selon l'une des revendications précédentes, **caractérisé en ce que** lesdits organes (9,16) définissent chacun une demi sphère dont l'une (9) est fixée à une extrémité (8) du corps (2) de l'instrument, l'autre demi sphère (16) étant fixée à une extrémité d'un bras (10) mobile par rapport audit corps (2).

6. Instrument selon l'une des revendications précédentes, **caractérisé en ce que** l'un (16) des organes est pourvu d'un décrochement (17) formant un passage pour la tête (9) d'une tique (20).

7. Instrument selon la revendication 6, **caractérisé en ce que** le générateur piézoélectrique (21) alimente deux conducteurs électriques (220) disposés de part et d'autre du passage (17), les conducteurs (220) étant adaptés pour réaliser un arc électrique lorsque les organes (9, 16) forment le volume clos (18).

8. Instrument selon l'une des revendications 1 ou 2, **caractérisé en ce que** ledit générateur piézoélectrique (21) alimente, dans chaque organe (9,16), au moins un conducteur électrique (220) disposé sur la face interne, les conducteurs (220) étant adaptés pour réaliser un arc électrique lorsque les organes (9,16) forment le volume clos (18).

9. Instrument selon l'une des revendications précédentes, **caractérisé en ce qu'**au moins un organe (9,16) est réalisé en un matériau transparent.

10. Instrument selon la revendication 9, **caractérisé en ce que** ledit organe transparent forme une loupe.

11. Instrument selon l'une des revendications précédentes, **caractérisé en ce que** le corps (2) de l'instrument porte l'un (9) desdits organes alors que l'autre organe est porté par un bras (10) monté mobile sur le corps et **en ce que** le corps est équipé d'un moyen (24) de rappel en position du bras mobile (10) vers une position où les organes définissent ensemble le volume clos (18) de maintien de la tique (20).

## Claims

1. Instrument for removing ticks that are fixed to the skin of an animal or human being, said instrument containing a body (2) and two parts movable in relation to one another, said parts (2, 10) each containing a component (9, 16) for defining an enclosed space (18) with the other component (16, 9) to hold the body and head (19) of a tick (20) without compression, said space (18) being supplied with a means of lethal neutralisation (220) for said tick (20) connected to a piezoelectric generator (21) for supplying an electric voltage to the means of neutralisation to cause a lethal electric shock to the tick, **characterised in that** the piezoelectric generator (21) is inserted into the body (2) of the instrument and is actuated by displacement of a cap (23) covering one end of the body (2) of the instrument.

2. Instrument according to claim 1, **characterised in that** the electric voltage supplied to the means of neutralisation (220) by the piezoelectric generator (21) is at least 17,000 volts.

3. Instrument according to either of the previous claims, **characterised in that** the piezoelectric generator (21) is equipped with a device to prevent a drop in the electric voltage supplied to the means of neutralisation (220) over time.

4. Instrument according to claim 3, **characterised in that** said device comprises an electric capacitor discharging into an electric coil to amplify and linearise the voltage supplied by the piezoelectric generator (21).

5. Instrument according to any of the previous claims, **characterised in that** said components (9, 16) each define a hemisphere, one of which (9) is fixed to one end (8) of the body (2) of the instrument, the other hemisphere (16) being fixed to one end of an arm (10) which is movable in relation to said body (2).

6. Instrument according to any of the previous claims, **characterised in that** one of the components (16) has a break (17) forming an opening for the head (9) of a tick (20).

7. Instrument according to claim 6, **characterised in that** the piezoelectric generator (21) feeds two electric conductors (220) arranged on either side of the opening (17), the conductors (220) being able to produce an electric arc when the components (9, 16) form the enclosed space (18).

8. Instrument according to either of claims 1 or 2, **characterised in that** said piezoelectric generator (21) feeds at least one electric conductor (220) in each component (9, 16), located on the inner face, said conductors (220) being able to produce an electric arc when the components (9, 16) form the enclosed space (18).

9. Instrument according to any of the previous claims, **characterised in that** at least one component (9, 16) is manufactured in a transparent material.

10. Instrument according to claim 9, **characterised in that** said transparent component forms a magnifying glass.

11. Instrument according to any of the previous claims, **characterised in that** the body (2) of the instrument holds one of the said components (9) whilst the other component is held by an arm (10) mounted movably on the body and **in that** the body is equipped with a means (24) of position return of the movable arm (10) to a position in which the components together define the enclosed space (18) to hold the tick (20).

## Patentansprüche

1. Instrument, das das Herausziehen von auf der Haut eines Tieres oder eines Menschen festgesetzten Zecken gestattet, wobei das Instrument einen Körper (2) und zwei zueinander bewegliche Teile aufweist und die Teile (2, 10) jedes ein Organ (9, 16) umfassen, das angepasst ist, um mit dem anderen Organ (16, 9) ein geschlossenes Volumen (18) zum Halten des Körpers und des Kopfes (19) einer Zecke (20) ohne Druckbelastung zu begrenzen, wobei das Volumen (18) mit einem Mittel (220) zum letalen Neutralisieren der Zecke (20) versehen ist, das an einen piezoelektrischen Generator (21) angeschlossen ist, der geeignet ist, an das Mittel zum Neutralisieren eine elektrische Spannung zu liefern, die einen letalen elektrischen Schock für die Zecke hervorruft, **dadurch gekennzeichnet, dass** der piezoelektrische Generator (21) in den Körper (2) des Instruments eingefügt ist und dass er durch die Verschiebung einer Kappe (23) betätigt wird, die auf ein Ende des Körpers (2) des Instruments aufgesetzt ist.

2. Instrument nach Anspruch 1, **dadurch gekennzeichnet, dass** die elektrische Spannung, die von dem piezoelektrischen Generator (21) an das Mittel zum Neutralisieren geliefert wird, mindestens 17000 Volt beträgt.

3. Instrument nach einem der vorhergehenden Ansprüche, **dadurch gekennzeichnet, dass** der piezoelektrische Generator (21) mit einer Vorrichtung ausgerüstet ist, die geeignet ist, einen zeitlichen Abfall der elektrischen Spannung, die an das Mittel (220) zum Neutralisieren geliefert wird, zu vermeiden.

4. Instrument nach Anspruch 3, **dadurch gekennzeichnet, dass** die Vorrichtung eine elektrische Kapazität umfasst, die sich in eine elektrische Spule entlädt, die angepasst ist, die von dem piezoelektrischen Generator (21) gelieferte Spannung zu verstärken und zu linearisieren.

5. Instrument nach einem der vorhergehenden Ansprüche, **dadurch gekennzeichnet, dass** die Organe (9, 16) jeweils eine Halbkugel begrenzen, von denen eine (9) an einem Ende (8) des Körpers (2) des Instruments befestigt ist und die andere Halbkugel (16) an dem Ende eines in Bezug auf den Körper (2) beweglichen Arm (10) befestigt ist.

6. Instrument nach einem der vorhergehenden Ansprüche, **dadurch gekennzeichnet, dass** eines (16) der Organe mit einem Absatz (17) versehen ist, der einen Durchgang für den Kopf (9) einer Zecke (20) bildet.

7. Instrument nach Anspruch 6, **dadurch gekennzeichnet, dass** der piezoelektrische Generator (21) zwei elektrische Leiter (220) versorgt, die beidseitig des Durchgangs (17) angeordnet sind, wobei die Leiter (220) angepasst sind, einen Lichtbogen zu erzeugen, wenn die Organe (9, 16) das geschlossene Volumen (18) bilden.

8. Instrument nach einem beliebigen der Ansprüche 1 oder 2, **dadurch gekennzeichnet, dass** der piezoelektrische Generator (21) in jedem Organ (9, 16) mindestens einen elektrischen Leiter (220) versorgt, der auf der Innenfläche angeordnet ist, wobei die Leiter (220) angepasst sind, einen Lichtbogen zu erzeugen, wenn die Organe (9, 16) das geschlossene Volumen (18) bilden.

9. Instrument nach einem der vorhergehenden Ansprüche, **dadurch gekennzeichnet, dass** mindestens ein Organ (9, 16) aus einem transparenten Material hergestellt ist.

10. Instrument nach Anspruch 9, **dadurch gekennzeichnet, dass** das transparente Organ eine Lupe bildet.

11. Instrument nach einem beliebigen der vorhergehenden Ansprüche, **dadurch gekennzeichnet, dass** der Körper (2) des Instruments eines (9) der Organe trägt, während das andere Organ von einem Arm (10) getragen wird, der beweglich an dem Körper befestigt ist, und dass der Körper mit einem Mittel (24) zum Rückstellen in Position des beweglichen Arms zu einer Position, bei der die Organe zusammen das geschlossene Volumen (18) zum Halten der Zecke (20) bilden.
